# NEUE EUROPÄISCHE PATENTSCHRIFT

(11) **EP 0 988 392 B2**
(45) Veröffentlichungstag und Bekanntmachung des Hinweises auf die Entscheidung über den Einspruch: **05.06.2013**
(45) Hinweis auf die Patenterteilung: 05.04.2006
(21) Anmeldenummer: 98933615.1
(22) Anmeldetag: 15.06.1998
(51) Int. Cl.: C12P 5/02, C12M 1/107

(54) **VERFAHREN UND VORRICHTUNG ZUR BIOGASGEWINNUNG**
METHOD AND DEVICE FOR OBTAINING BIO-GAS
PROCEDE ET DISPOSITIF DE PRODUCTION DE BIOGAZ

(30) Priorität: 18.06.1997 DE 19725823
(43) Veröffentlichungstag der Anmeldung: 29.03.2000
(73) Patentinhaber: STRABAG Umweltanlagen GmbH, 01069 Dresden (DE)
(72) Erfinder: LANGHANS, Gerhard, D-01159 Dresden (DE)
(74) Vertreter: Wasmuth, Rolf
(86) Internationale Anmeldenummer: PCT/EP1998/003586
(87) Internationale Veröffentlichungsnummer: WO 1998/058070

(56) Entgegenhaltungen:
- CH-A- 316 767
- DD-A- 255 722
- DE-A- 2 207 920
- DE-A- 3 335 265
- DE-A- 4 322 738
- DE-A- 19 624 268
- US-A- 5 500 123
- DR. GERD REINHOLD: 'Untersuchungen zur grosstechnischen Erzeugung und Verwertung von Biogas bei Berücksichtigung der Substratveränderungen', 15 April 1998
- REINHOLD ET AL.: 'Einflussfaktoren auf die Effektivität landwirtschaftlicher' AGRARTECHNIK 1987, Seiten 454 - 456
- KOOPERATIONSVERBAND ORGANISHCE DÜNGUNG "VERGÄRUNGSANALAGE ALS CO-FERMENTATION VON RINDER- UND SCHWEINEGÜLLE"
- VEG TIERZUCHT NORDHAUSEN: 'GVA - Anfahrprojekt' DOKUMENTATION FÜR INBETRIEBSETZUNG UND ERPROBUNG DER BIOGASANLAGE HIMMELGARTEN 30 November 1983,
- TECHNOLOGISCHES GESAMTSCHEMA DER BIOGASANLAGE HIMMELGARTEN 28 November 2011,
- VOLLMER, GERD- RAINER, PROF. DR. ERKLÄRUNG DES ZUSTÄNDIGEN PROJEKTBETREUERS DER BIOGASANLAGE HIMMELGARTEN 12 Dezember 2011,
- LANGHANS, G.: 'Manure and biowaste digestion in Germany' THE 1ST INTERNATIONAL CONFERENCE ON GREENHOUSE GASES AND ANIMAL AGRICULTURE GGAA2001 07 November 2001, Seiten 120 - 131
- 'Biogasanlage Behringen', 10 November 1995 Seite 7
- ' Verfahrensbeschreibung der Biogasanlage', 10 November 1995, BEHRINGEN Seiten 1 - 4
- ' Verfahrenbeschreibung der Biogasanlage', 10 November 1995, BEHRINGEN vol. ' Dokument PMC 0002K', Seiten 9 - 19

## Beschreibung

Die Erfindung betrifft ein Verfahren zur Biogasgewinnung durch biologische Behandlung eines Gärmediums in einem Gärreaktor, bei dem dem Gärmedium zur Unterdrückung der Bildung von unerwünschtem Schwefelwasserstoff (H₂S) ein einen Elektronenakzeptor enthaltendes Medium, insbesondere sauerstoffenthaltendes Gas oder Gasgemisch, z. B. Luft und/oder Nitrat und/oder Nitrit, zugeführt wird, sowie eine Vorrichtung zur Durchführung des Verfahrens.

Bei der Gewinnung von Biogas werden organische Substanzen enthaltende Substrate, z. B. Klärschlämme, Gülle oder Naßmüll, in einem Behälter unter weitgehendem Luftabschluß gehalten und gegebenfalls umgerührt. Dabei wandeln die in den Substraten enthaltenen Mikroorganismen die organischen Substanzen teilweise in gasförmige Stoffe um. Dieser als Faulung bezeichnete Vorgang wird üblicherweise in als Faulbehälter ausgebildeten Bioreaktoren durchgeführt, die auch als Gärreaktoren bezeichnet werden. Je nach Beschaffenheit der Substrate und des Bioreaktorbetriebes ergeben sich leicht unterschiedliche Zusammensetzungen des Biogases. Typischerweise enthält das Biogas ca. 70 Vol.-% CH₄ und ca. 30 Vol.-% CO₂. Weisen die Substrate auch Schwefelverbindungen auf, was bei Klärschlämmen und Naßmüll meist der Fall ist, werden diese durch die Mikroorganismen zu Schwefelwasserstoff (H₂S) abgebaut, der sich letztlich mit einer Konzentration von bis zu 1 Vol.-% im Biogas wiederfindet. Da Schwefelwasserstoff toxisch und korrosiv wirkt, muß zur Vermeidung von Umweltschäden und Schäden in nachgeschalteten Anlagen, z. B. Leitungen und Gasmotoren, der Schwefelwasserstoffgehalt des Biogases verringert werden.

Nach dem Stand der Technik wird der Schwefelwasserstoffgehalt des Biogases in dem Bioreaktor nachgeschalteten Reinigungsstufen, die z. B. als Wäsche, Adsorptionseinheit oder biologische Entschwefelungsanlage ausgebildet sein können, auf einen vertretbaren Wert reduziert. Aufgrund der notwendigen zusätzlichen Reinigungsstufe fallen hohe Investitionskosten an und der Platzbedarf für die gesamte Biogasanlage vergrößert sich. Bei einer Nachreinigung in der Gasphase fallen darüberhinaus meist Abprodukte an, bspw. Fe-Sulfid-Pellets, Schwefelblüte, Schwefelsäure, etc., die entsorgt werden müssen.

Aus der DD 226 552 A1 ist ein Verfahren zur Reduzierung des Schwefelwasserstoffgehalts im Biogas bekannt, bei dem Eisenhydroxid in suspendierter Form dem zu behandelnden Schlamm zugegeben und mit diesem vermischt wird. Dadurch soll der Schwefelwasserstoff chemisch gebunden werden. Es ist auch bekannt, Eisenchlorid dem zu behandelnden Gärmedium zuzudosieren. Derartige mit einer Chemikaliendosierung arbeitenden Verfahren sind allerdings aufgrund der Chemikalienzugabe zum Gärmedium problematisch. Beispielsweise kann sich der Eintrag korrosiver Chloridionen negativ auf die Haltbarkeit des Gärreaktors auswirken.

Mit der EP 0 143 149 B1 ist auch schon vorgeschlagen worden, bereits die Bildung von Schwefelwasserstoff im Bioreaktor zu unterdrücken. Dabei wird eine solche Menge an Sauerstoff mit dem Frischschlamm oder dem Wasser in den Bioreaktor eingebracht, daß im sich bildenden Biogas ein Restsauerstoffgehalt von 0,01 bis 3,0 Vol.-% enthalten ist. Hierzu ist eine aufwendige Steuerung notwendig, um sicherzustellen, daß einerseits der Schwefelwasserstoffgehalt im Biogas ausreichend verringert wird und andererseits der Sauerstoffgehalt im Bioreaktor nicht so hoch wird, daß eine zu starke Behinderung der Biogasbildung durch die toxische Wirkung des Sauerstoffs auf die menthanbildenden Bakterien eintritt.

Die bisherigen Lösungen zur Unterdrückung der H₂S-Bildung durch Hemmung der H₂S-erzeugenden Mikroflora gehen von einer Luftdosierung in den Gärzulauf oder von punktuellem Lufteintrag in den Gärreaktor aus. Die Anlagenpraxis und eigene Untersuchungen zu diesem Problem zeigten, daß aufgrund der hohen Sauerstoffverbrauchsgeschwindigkeiten Zulauf- oder punktuelle Dosierungen in der Regel nicht genügen, da der Sauerstoff schon nach kurzer Distanz biochemisch umgesetzt ist, und somit die Störung der H₂S-Bildner in dem großräumigen Gärreaktor nicht ausreicht, um einen signifikanten Effekt zu erzielen.

Man versucht, sich dagegen durch Luftdosierung im Überschuß zu helfen, was jedoch ein erhöhtes Sicherheitsrisiko birgt und durch den dann hohen Stickstoffinertanteil im Biogas zu einer kalorischen Qualitätsminderung führt.

Des weiteren kann versucht werden, durch entsprechend flächendeckende Luftverteilungseinrichtungen am Fermenterboden eine bessere Beaufschlagung im Gesamtreaktor zu erreichen. Für trockensubstanzreiche Abfall- und Schlammgärsuspensionen ist die langzeitliche Betriebssicherheit solcher Verteileinrichtungen jedoch in Frage gestellt.

Der Erfindung liegt die Aufgabe zugrunde, ein Verfahren der eingangs genannten Art sowie eine Vorrichtung zur Durchführung des Verfahrens zur Verfügung zu stellen, mit denen gewährleistet wird, daß das den Gärreaktor verlassende Biogas weitgehend frei von Schwefelwasserstoff ist und daß keine wesentliche Beeinträchtigung der Biogasbildung stattfindet, wobei die geschilderten Nachteile des Standes der Technik vermieden werden.

Diese Aufgabe wird erfindungsgemäß verfahrensseitig dadurch gelöst, daß das gesamte im Gärreaktor enthaltene Gärmedium durch eine mit dem den Elektronenakzeptor enthaltenden Medium beaufschlagte Zone geführt wird, wobei eine ausreichende Kontaktzeit zwischen dem den Eektronenakzeptor enthaltenden Medium und dem Gärmedium eingestellt wird, um die H₂S-Bildung im Gärmedium zumindest soweit zu unterdrücken, daß im Biogas keine wesentlichen H₂S-Anteile vorhanden sind.

Als elektronenakzeptorenthaltendes Medium wird zweckmäßigerweise Luft oder ein anderes sauerstoffhaltiges Gas oder Gasgemisch verwendet. Eine andere Möglichkeit besteht darin, als Elektronenakzeptor Nitrit oder Nitrat einzusetzen. Auch eine Kombination verschiedener Elektronenakzeptoren ist denkbar.

Das Wesen der Erfindung läßt sich am einfachsten im Falle der Verwendung von Luft als elektronenakzeptorenthaltendes Medium folgendermaßen darstellen:

Die genannten Probleme des Standes der Technik werden mit der Erfindung in der Weise gelöst, daß nicht eine weitgehend homogene Luftverteilung im gesamten Gärraum versucht wird, sondem der gesamte Gärreaktorinhalt definiert durch eine sauerstoffhaltige Zone mit ausreichender Kontaktzeit zwischen Gas und Gärmedium gefördert wird. Auf diese Weise wird eine technisch einfache und rückstandsfreie Unterdrückung der H₂S-Bildung im Gärmedium reproduzierbar erreicht. Mit dem erfindungsgemäßen Verfahren wird die Unterdrückung der H₂S-Bildung im Gärmedium anlagentechnisch betriebsstabil gewährleistet.

Zweckmäßigerweise wird das gesamte im Gärreaktor enthaltene Gärmedium mehrmals, bevorzugt mindestens zweimal pro Stunde, durch die Zone geführt Außerdem wird das elektronenakzeptorenthaltende Medium vorzugsweise in solchen Mengen in die Zone dosiert, daß das Gärmedium während der Passage der Zone ausreichenden Kontakt mit dem elektronenakzeptorenthaltenden Medium erhält, um die H₂S- Bildung im Gärmedium zu unterdrücken.

Gemäß einer bevorzugten Ausführungsform der Erfindung wird die Kontaktzeit zwischen dem elektronenakzeptorenthaltenden Medium und dem Gärmedium in der Zone und/oder die Menge des in die Zone dosierten elektronenakzeptorenthaltenden Mediums so eingestellt, daß das elektronenakzeptorenthaltende Medium biochemisch soweit abgebaut wird, daß im Biogas keine prozeßbeeinträchtigenden Anteile an elektronenakzeptorenthaltendem Medium mehr vorhanden sind. Im Falle der Verwendung von Luft oder einem sauerstoffenhaltenden Gas oder Gasgemische als elektronenakzeptorenthaltendem Medium wird auf diese Weise erreicht, daß der Sauerstoff biochemisch soweit abgebaut wird, daß im Biogas keine prozeßbeeinträchtigenden Sauerstoffanteile mehr vorhanden sind. Durch diese Verfahrensführung wird sichergestellt, daß im Gärreaktor keine Beeinträchtigung der anaeroben Behandlung der organischen Substanzen und damit der Biogasgewinnung erfolgt.

Bei Verwendung von Luft als elektronenaktzeptorenthaltendem Medium wird außerdem die der Zone zugeführte Luftmenge pro Zeiteinheit vorzugsweise so eingestellt, daß der aus dem Stickstoffgehalt der Luft im Biogas resultierende Stickstoffgehalt zu keiner wesentlichen Qualitätsminderung des Biogases hinsichtlich einer kalorischen Nutzung führt. Eine Verwertung des Biogases bspw. als Brenngas ist dadurch uneingeschränkt möglich.

Eine besonders bevorzugte Ausführungsform der Erfindung sieht vor, das im Gärreaktor gebildete Biogas als Treibgas zu verwenden, um das Gärmedium durch die Zone zu fördem. Ist die Zone bspw. als Leitrohr ausgebildet, so wird Biogas in den Leitrohrinnenraum gepumpt. Infolge der Gemischdichteabsenkung im Leitrohr und der Gasauftriebskraft wird das Gärmedium von unten nach oben durch das Leitrohr gefördert, Dabei werden zweckmäßigerweise die hydraulischen Verhältnisse durch Wahl der Leitrohrgeometrie und des eingepreßten Biogasstromes dergestalt eingestellt, daß der gesamte Gärreaktorinhalt mindestens zweimal pro Stunde durch das Leitrohr gepumpt wird.
Die Erfindung betrifft femer eine Vorrichtung zur Biogasgewinnung mit einem Gärreaktor zur Aufnahme von Gärmedium und einer Ableitung für Biogas. Vorrichtungsseitig wird die gestellte Aufgabe dadurch gelöst, daß im Innenraum des Gärreaktors eine Leiteinrichtung angeordnet ist, wobei in der Leiteinrichtung oder in Nähe eines offenen Endes der Leiteinrichtung mindestens eine Zuleitung für ein elektronenakzeptorenthaltendes Medium endet, sowie Mittel zum Fördern des gesamten im Gärreaktor enthaltenden Gärmediums durch die Leiteinrichtung vorgesehen sind.

Der Gärreaktor ist also im wesentlichen als Schlaufenreaktor mit innenliegender Schlaufe in Form einer Leiteinrichtung, die bspw. als Leitrohr ausgebildet sein kann, gestaltet. Vorzugsweise ist die Leiteinrichtung als im wesentlichen vertikal und zentrisch im Gärreaktor angeordnetes Leitrohr ausgebildet.

Zum Einpreßen von Biogas als Treibgas endet vorzugsweise eine von der Biogasableitung abzweigende Biogaszweigleitung in der Leiteinrichtung oder in Nähe eines offenen Endes der Leiteinrichtung.

Gemäß einer weiteren bevorzugten Ausführungsform der Erfindung ist die Geometrie der Leiteinrichtung so gewählt, daß eine ausreichende Kontaktzeit zwischen dem Gärmedium und dem elektronenakzeptorenthaltenden Medium gewährleistet ist, um eine H₂S-Bildung im Gärmedium zu unterdrücken.

Weiterhin werden vorzugsweise die Geometrie der Leiteinrichtung, die Größe des Gärreaktors und die Dimensionierung der Biogaszweigleitung so gewählt, daß das gesamte im Gärreaktor enthaltene Gärmedium mindestens zweimal pro Stunde durch die Leiteinrichtung geführt werden kann.

Eine weitere Ausgestaltung der erfindungsgemäßen Vorrichtung sieht vor, daß die Leiteinrichtung beheizbar ausgeführt ist. Hierzu ist die Leiteinrichtung zweckmäßigerweise als Leitrohr ausgebildet, das einen Doppelmantel aufweist Der Doppelmantel ist mit einer Zuführung und einer Abführung für Heizwasser versehen.

Bei dieser Ausgestaltung der Erfindung werden mehrere Effekte, die eine prozeßstabile Gewinnung von im wesentlichen H₂S-freiem Biogas ermöglichen, gleichzeitig erzielt:

Durch Umwälzung des Gärmediums über die als Leitrohr ausgebildete innere Schlaufe des Gärreaktors wird der gesamte Gärreaktorinhalt homogenisiert Aufgrund der Dosierung eines elektronenakzeptorenthaltenden Mediums, z. B. Luft, wird gleichzeitig die H₂S-Bildung im Gärreaktor unterdrückt. Außerdem wird der Gärreaktorinhalt durch die Beheizung des Leitrohres auf einer für die biologische Behandlung des Gärmediums opitmalen Betriebstemperatur gehalten.

Bei vertikaler, zentrischer Ausrichtung des Leitrohres im Gärreaktor wird noch ein zusätzlicher Effekt erreicht:

Das vom unteren Ende des Leitrohres angesaugte Gärmedium wird vorzugsweise durch Einpreßen von Biogas zum oberen Ende des Leitrohres gepumpt und wird am oberen Ende des Leitrohres vertikal nach oben in das umgebende Gärmedium ausgestoßen. Durch den so bewirkten zentrischen Oberflächenschwall über dem Leitrohr wird eine Schwimmdeckenzerstörung im Gärreaktor erreicht, was sich wiederum positiv auf die Prozeßstabiltät auswirkt

Die Erfindung bietet eine ganze Reihe von Vorteilen:

Im Gegensatz zu Verfahren nach dem Stand der Technik ist für die H₂S-Unterdrückung keine Chemikaliendosierung notwendig. Außerdem ist keine Abproduktentsorgung erforderlich. Mit der Erfindung wird eine besonders hohe Prozeßstabilität mit technisch einfachen Mitteln erreicht. Eine Nachrüstung bestehender Biogasanlagen ist mit minimalem Aufwand möglich. Im einfachsten Fall muß lediglich eine Luftdosierung in einem im Gärreaktor eingesetzten Leitrohr-homogenisierungssystem installiert werden.

Im folgenden soll die Erfindung anhand eines in den Figuren schematisch dargestellten Ausführungsbeispiels näher erläutert werden.

Es zeigen
- Figur 1: ein Fließbild einer Biogasanlage mit integrierter H₂S-Unterdrückung
- Figur 2: einen Vergleich der Betriebsergebnisse von Gärreaktoren mit und ohne Luftdosierung in den Gärraum

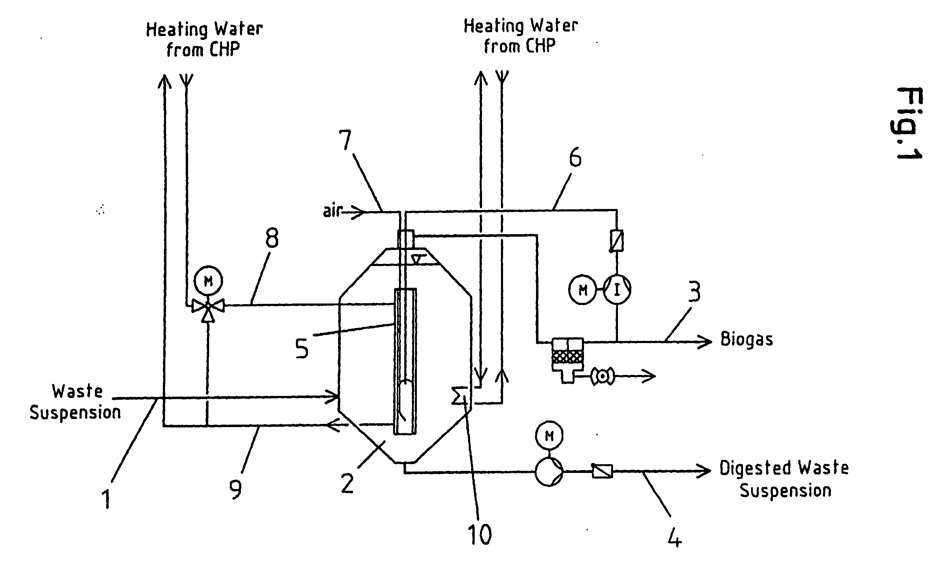

In Figur 1 ist beispielhaft eine Anlage zur Vergärung von Naßmüll dargestellt Der Naßmüll wird in in der Figur nicht gezeigten Vorbehandlungsschritten so aufbereitet, daß Pulpe bzw. Hydrolisat entstehen. Die Pulpe bzw. das Hydrolisat werden als Gärmedium über Leitung 1 dem Gärreaktor 2 zugeführt. Im Gärreaktor 2 wird die Methanisierung der Pulpe bzw. des Hydrolisats durchgeführt. Hierzu wird der Gärreaktor 2 unter anaeroben Bedingungen gehalten und der Gärreaktorinhalt umgewältzt. Die in der gärenden Pulpe bzw. dem Hydrolisat enthaltene anaerobe Biomasse wandelt die organischen Substanzen teilweise in CO₂ und CH₄ um. Das anfallende Biogas wird über Leitung 3 aus dem Gärreaktor 2 abgezogen. Die flüssigen und/oder festen Anteile des vergärten Naßmülls werden über Leitung 4 aus dem Gärreaktor 2 abgeführt

Da die Pulpe bzw. das Hydrolisat auch Schwefelverbindungen enthalten, würde ohne weitere Maßnahmen auch H₂S gebildet, das sich schließlich im Biogas wiederfinden würde. Um die unerwünschten H₂S-Anteile im Biogas zu minimieren, wird im Gegensatz zum Stand der Technik, bei dem eine weitgehend homogene Luffirerteilung im gesamten Gärraum versucht wird, der gesamte Gärreaktorinhalt definiert durch eine sauerstoffhaltige Zone 5 mit ausreichender Kontaktzeit zwischen sauerstoffhaltigem Gas und Gärmedium gefördert Zu diesem Zweck ist der Gärreaktor 2 als Schlaufenreaktor mit innenliegender Schlaufe in Form eines zentrisch, vertikal angeordneten Leitrohres 5, das als sauerstoffhaltige Zone fungiert, gestaltet. Dabei dient in den unteren Teil des Leitrohrinnenraumes gepumptes Biogas, das über eine Biogaszweigleitung 6 von der Biogasableitung 2 abgezweigt wird, als Treibgas. Infolge der Gemischdichteabsenkung im Leitrohr 5 und der Gasauftriebkraft wird das Gärmedium von unten nach oben durch das Leitrohr 5 gefördert. Dabei werden die hydraulischen Verhältnisse durch Wahl der Leitrohrgeometrie und des eingepreßten Biogasstromes dergestalt eingestellt, daß der gesamte Gärreaktorinhalt mindestens zweimal pro Stunde durch das Leitrohr 5 gepumpt wird. In den inneren Aufstrom des Leitrohres 5 wird Luft mittels einer Luftzuleitung 7 in solchen Mengenverhältnissen dosiert, daß das Gärmedium während der Passage des Leitrohres 5 ausreichend Sauerstoffkontakt bekommt, um die H₂S-Bildung in ihren Stoffwechselprozeßen in der gewünschten Weise zu limitieren. Gleichzeitig wird der Sauerstoff biochemisch soweit abgebaut, daß im Biogas keine prozeßbeeinträchtigenden Sauerstoffanteile mehr vorhanden sind. Der Luftbedarf kann dabei so minimiert werden, daß der Stickstoff im Biogas nicht zu einer Gasqualitätsminderung für die weitere kalorische Nutzung führt.

Zur Aufrechterhaitung einer für die biologische Behandlung des Gärmediums optimalen Betriebstemperatur ist das Leitrohr 5 beheizbar ausgebildet. Hierzu ist das Leitrohr 5 mit einem doppelwandigen Mantel versehen, der eine Zuführung 8 und eine Abführung 9 für Heizwasser aufweist. Zusätzlich kann der Gärreaktorinhalt mittels eines Wärmetauschers 19, der von Heizwasser durchflossen wird, temperiert werden.

Durch die Verwendung eines zentrischen, vertikalen Leitrohres 5 im Gärreaktor 2 als sauerstoffhaltige Zone werden mehrere Effekte gleichzeitig erzielt

Einerseits wird aufgrund der Luftdosierung im Leitrohr 5 eine zuverlässige H₂S-Unterdrückung erreicht. Andererseits wird der Gärreaktorinhalt durch Umwälzung über die innere Schlaufe des Gärreaktors 2 homogenisiert. Darüber hinaus wird eine Gärreaktortemperierung mittels durch den Leitrohrdoppelmantel gepumptem Heizwasser bewirkt Schließlich wird aufgrund des Austritts von Gärmedium aus dem oberen Ende des Leitrohres 5 ein zentrischer Obenflächenschwall über dem Leitrohr 5 erzeugt, der zu einer Schwimmdeckenzerstörung im Gärreaktor 2 führt.

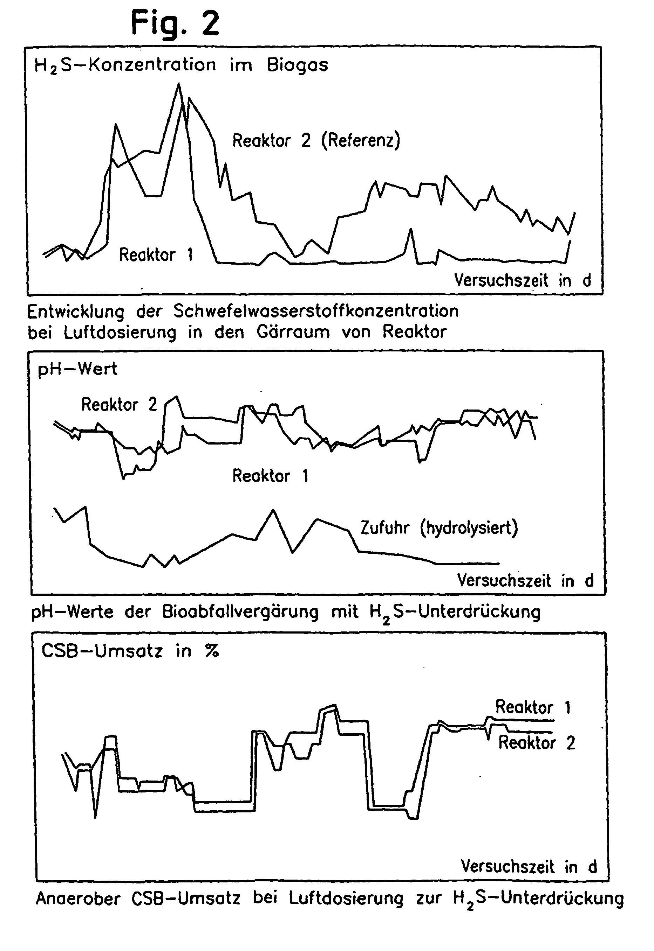

Die beschriebene Zirkulation des Gärmediums reicht aus, die H₂S-Bildner des gesamten Gärraumes sicher in der gewünschten Weise zu beeinträchtigen. Die Methanbildung aus der Gärreaktion wird dabei nicht beeinträchtigt, wie die in Figur 2 dargestellten Messungen beweisen.

In Figur 2 sind die Betriebsergebnisse eines Gärreaktors mit Luftdosierung in den Gärraum (Reaktor 1) im Vergleich mit denen eines herkömmlichen Gärreaktors ohne Luftdosierung (Reaktor 2) gezeigt.

## Patentansprüche

1. Verfahren zur Biogasgewinnung durch biologische Behandlung eines Gärmediums in einem Gärreaktor, bei dem dem Gärmedium zur Unterdrückung der Bildung von unerwünschtem Schwefelwasserstoff (H₂S) ein einen Elektronenakzeptor enthaltendes Medium, insbesondere sauerstoffenthaltendes Gas oder Gasgemisch, z. B. Luft und/oder Nitrat und/oder Nitrit, zugeführt wird,
**dadurch gekennzeichnet, dass** in dem Gärreaktor (2), der als Schlaufenrektor mit innenliegender Schlaufe in Form eines Leitrohres (5) ausgeführt ist, das gesamte im Gärreaktor (2) enthaltene Gärmedium durch das mit dem den Elektronenakzeptor enthaltenden Medium beaufschlagte Leitrohr (5) geführt wird, wobei eine ausreichende Kontaktzeit zwischen dem den Elektronenakzeptor enthaltenden Medium und dem Gärmedium eingestellt wird, um die H₂S-Bildung im Gärmedium zumindest soweit zu unterdrücken, dass im Biogas keine wesentlichen H₂S-Anteile vorhanden sind, und dass das gesamte im Gärreaktor (2) enthaltene Gärmedium mehrmals, vorzugsweise mindestens zweimal pro Stunde, durch das Leitrohr (5) geführt wird, und dass durch die Umwälzung des Gärmediums über die als Leitrohr (5) ausgebildete innere Schlaufe des Gärreaktors (2) der gesamte Reaktorinhalt homogenisiert wird.

2. Verfahren nach Anspruch 1,
**dadurch gekennzeichnet, dass** das den Elektronenakzeptor enthaltende Medium in solchen Mengen in das Leitrohr (5) dosiert wird, dass das Gärmedium während der Passage des Leitrohrs (5) ausreichend Kontakt mit dem den Elektronenakzeptor enthaltenden Medium erhält, um die H₂S-Bildung im Gärmedium zu unterdrücken.

3. Verfahren nach Anspruch 1 oder 2,
**dadurch gekennzeichnet, dass** die Kontaktzeit zwischen dem den Elektronenakzeptor enthaltenden Medium und dem Gätmedium im Leitrohr (5) und/ oder die Menge des in das Leitrohr (5) dosierten, den Elektronenakzeptor enthaltenden Mediums so eingestellt wird, dass das den Elektronenakzeptor enthaltende Medium biochemisch soweit abgebaut wird, dass im Biogas keine prozessbeeinträchtigenden Anteile an dem den Elektronenakzeptor enthaltenden Medium mehr vorhanden sind.

4. Verfahren nach einem der Ansprüche 1 bis 3,
**dadurch gekennzeichnet, dass** bei Verwendung von Luft als den Elektronenakzeptor enthaltendes Medium die dem Leitrohr (5) zugeführte Luftmenge pro Zeiteinheit so eingestellt wird, dass der aus dem Stickstoffgehalt der Luft im Biogas resultierende Stickstoffgehalt zu keiner wesentlichen Qualitätsminderung des Biogases hinsichtlich einer kalorischen Nutzung führt.

5. Verfahren nach einem der Ansprüche 1 bis 4,
**dadurch gekennzeichnet, dass** das Gärmedium mittels eines Treibgases, das vorzugsweise aus dem Biogas besteht, durch das Leitrohr (5) gefördert wird.

6. Vorrichtung zur Biogasgewinnung mit einem Gärreaktor zur Aufnahme von Gärmedium und einer Ableitung von Biogas,
**dadurch gekennzeichnet, dass** im Innenraum des Gärreaktors (2)
ein Leitrohr (5) angeordnet ist, und dass es sich um einen Gärreaktor (2) handelt, der als Schlaufenreaktor mit innenliegender Schlaufe in Form eines Leitrohres (5) ausgeführt ist, wobei im Leitrohr (5) oder in Nähe eines offenen Endes des Leitrohrs (5) mindestens eine Zuleitung (7) für ein einen Elektronenakzeptor enthaltendes Medium endet, sowie Mittel zum Fördern des gesamten im Gärreaktor (2) enthaltenen, durch diese Umwälzung homogenisierten Gännediums durch das Leitrohr (5) vorgesehen sind.

7. Vorrichtung nach Anspruch 6,
**dadurch gekennzeichnet, dass** das Leitrohr (5) vertikal und zentrisch im Gärreaktor (2) angeordnet ist.

8. Vorrichtung nach Anspruch 6 oder 7,
**dadurch gekennzeichnet, dass** in dem Leitrohr (5) oder in der Nähe eines offenen Endes des Leitrohrs (5) eine von der Biogasableitung (3) abzweigende Biogaszweigleitung (6) endet.

9. Vorrichtung nach einem der Ansprüche 6 bis 8,
**dadurch gekennzeichnet, dass** die Geometrie der Leiteinrichtung (5) so gewählt ist, dass eine ausreichende Kontaktzeit zwischen dem Gärmedium und dem den Elektronenakzeptor enthaltenden Medium gewährleistet ist, um eine H₂S-Bildung im Gärmedium im Wesentlichen zu unterdrücken.

10. Vorrichtung nach einem der Ansprüche 6 bis 9,
**dadurch gekennzeichnet, dass** die Geometrie des Leitrohrs (5), die Größe des Gärreaktors (2) und die Dimensionierung der Biogaszweigleitung (6) so gewählt sind, dass zur Homogenisierung des gesamten Gärreaktorinhaltes das gesamte im Gärreaktor (2) enthaltene Gärmedium mindestens zweimal pro Stunde durch das Leitrohr (5) gefördert werden kann.

## Claims

1. Process for obtaining biogas by biological treatment of a fermentation medium in a fermentation reactor, in which an electron acceptor-containing medium, in particular oxygen-containing gas or gas mixture, for example air and/or nitrate and/or nitrite, is fed into the fermentation medium to suppress the formation of unwanted hydrogen sulphide (H₂S),
**characterized in that**, in the fermentation reactor (2), which is configured as a loop reactor having an internal loop in the form of a conduit pipe (5), all the fermentation medium contained in the fermentation reactor (2) is passed through the conduit pipe (5) into which the electron acceptor-containing medium is fed, with a sufficient time of contact between the electron acceptor-containing medium and the fermentation medium being adjusted in order to suppress H₂S formation in the fermentation medium at least to the extent that negligible amounts of H₂S are present in the biogas, and that all the fermentation medium contained in the fermentation reactor (2) is passed several times, preferably at least twice, an hour through the conduit pipe (5), and that all the reactor contents are homogenized by the circulation of the fermentation medium via the inner loop, configured as a conduit pipe (5), of the fermentation reactor (2).

2. Process according to Claim 1,
**characterized in that** the electron acceptor-containing medium is metered into the conduit pipe (5) in amounts such that the fermentation medium maintains adequate contact with the electron acceptor-containing medium while passing through the conduit pipe (5) in order to suppress H₂S formation in the fermentation medium.

3. Process according to Claim 1 or 2,
**characterized in that** the time of contact between the electron acceptor-containing medium and the fermentation medium in the conduit pipe (5) and/or the amount of the electron acceptor-containing medium metered into the conduit pipe (5) is adjusted so that the electron acceptor-containing medium is broken down biochemically to such an extent that process-impairing amounts of the electron acceptor-containing medium are no longer present in the biogas.

4. Process according to one of Claims 1 to 3,
**characterized in that**, when air is used as the electron acceptor-containing medium, the amount of air fed into the conduit pipe (5) per unit time is adjusted so that the nitrogen content resulting in the biogas from the nitrogen content of the air results in negligible reduction in the quality of the biogas in terms of caloric utilization.

5. Process according to one of Claims 1 to 4,
**characterized in that** the fermentation medium is transported through the conduit pipe (5) by means of a propellant gas which preferably consists of the biogas.

6. Apparatus for obtaining biogas, having a fermentation reactor to receive fermentation medium and a biogas offtake line,
**characterized in that** a conduit pipe (5) is disposed in the interior of the fermentation reactor (2), and that it is a fermentation reactor (2) which is configured as a loop reactor having an internal loop in the form of a conduit pipe (5), with at least one feed line (7) for an electron acceptor-containing medium terminating in the conduit pipe (5) or in the vicinity of an open end of the conduit pipe (5), and means are provided for transporting all the fermentation medium, which is contained in the fermentation reactor (2) and is homogenized by this circulation, through the conduit pipe (5).

7. Apparatus according to Claim 6,
**characterized in that** the conduit pipe (5) is disposed vertically and centrally in the fermentation reactor (2).

8. Apparatus according to Claim 6 or 7,
**characterized in that** a biogas branch line (6) branching off the biogas offtake line (3) terminates in the conduit pipe (5) or in the vicinity of an open end of the conduit pipe (5).

9. Apparatus according to one of Claims 6 to 8,
**characterized in that** the geometry of the conduit arrangement (5) is chosen so that a sufficient time of contact between the fermentation medium and the electron acceptor-containing medium is ensured in order to substantially suppress H2S formation in the fermentation medium.

10. Apparatus according to one of Claims 6 to 9,
**characterized in that** the geometry of the conduit pipe (5), the size of the fermentation reactor (2) and the dimensions of the biogas branch line (6) are chosen so that all the fermentation medium contained in the fermentation reactor (2) can be transported at least twice an hour through the conduit pipe (5) to homogenize all the fermentation reactor contents.

## Revendications

1. Procédé pour la production de biogaz par traitement biologique d'un milieu de fermentation dans un réacteur de fermentation, dans lequel un milieu contenant un accepteur d'électrons, en particulier un gaz ou un mélange gazeux riche en oxygène, par exemple de l'air et/ou du nitrate et/ou du nitrite, est alimenté dans le milieu de fermentation pour inhiber la formation de sulfure d'hydrogène (H₂S) indésirable,
**caractérisé en ce que** dans le réacteur de fermentation (2) conçu comme un réacteur à boucle avec une boucle interne sous la forme d'un conduit directeur (5), la totalité du milieu de fermentation contenu dans le réacteur de fermentation (2) est guidée à travers le conduit directeur (5) sollicité par le milieu contenant l'accepteur d'électrons, un temps de contact suffisant étant réglé entre le milieu contenant l'accepteur d'électrons et le milieu de fermentation, afin d'inhiber la formation de H₂S dans le milieu de fermentation, du moins de manière à ce que le biogaz ne contienne pas de quantités substantielles de H₂S, et **en ce que** la totalité du milieu de fermentation contenu dans le réacteur de fermentation (2) est conduit plusieurs fois, de préférence au moins deux fois par heure, à travers le conduit directeur (5), et **en ce que** la circulation du milieu de fermentation dans la boucle interne du réacteur de fermentation (2) conçue comme conduit directeur (5) permet d'homogénéiser l'ensemble du contenu du réacteur.

2. Procédé selon la revendication 1,
**caractérisé en ce que** le milieu contenant l'accepteur d'électrons est dosé en quantités telles dans le conduit directeur (5), que le milieu de fermentation est suffisamment en contact avec le milieu contenant l'accepteur d'électrons lors de son passage dans le conduit directeur (5) pour inhiber la formation de H₂S dans le milieu de fermentation.

3. Procédé selon la revendication 1 ou 2,
**caractérisé en ce que** le temps de contact entre le milieu contenant l'accepteur d'électrons et le milieu de fermentation dans le conduit directeur (5) et/ou la quantité de milieu contenant l'accepteur d'électrons qui est dosée dans le conduit directeur (5) est/sont réglé(s) de manière à ce que le milieu contenant l'accepteur d'électrons se décompose biochimiquement jusqu'à la suppression dans le biogaz des quantités de milieu contenant l'accepteur d'électrons nuisibles au processus.

4. Procédé selon l'une des revendications 1 à 3,
**caractérisé en ce qu'**en cas d'utilisation d'air comme milieu contenant l'accepteur d'électrons, la quantité d'air apportée au conduit directeur (5) par unité de temps est réglée de manière à ce que la teneur en azote résultant de la teneur en azote de l'air dans le biogaz n'entraîne pas de diminution considérable de la qualité du biogaz en vue d'un usage calorique.

5. Procédé selon l'une des revendications 1 à 4,
**caractérisé en ce que** le milieu de fermentation est transporté à travers le conduit directeur (5) au moyen d'un gaz propulseur constitué de préférence du biogaz.

6. Dispositif pour la production de biogaz, avec un réacteur de fermentation pour la réception d'un milieu de fermentation et avec un conduit d'évacuation de biogaz, **caractérisé en ce qu'**un conduit directeur (5) est agencé dans l'espace intérieur du réacteur de fermentation (2), et **en ce qu'**il s'agit d'un réacteur de fermentation (2) conçu comme un réacteur à boucle avec une boucle interne sous la forme d'un conduit directeur (5), dans lequel au moins un conduit d'alimentation (7) pour un milieu contenant un accepteur d'électrons débouche dans le conduit directeur (5) ou à proximité d'une extrémité ouverte du conduit directeur (5), et des moyens sont prévus pour le transport de la totalité du milieu de fermentation contenu dans le réacteur de fermentation (2) et homogénéisé par cette circulation, à travers le conduit directeur (5).

7. Dispositif selon la revendication 6,
**caractérisé en ce que** le conduit directeur (5) est agencé verticalement et de façon centrée dans le réacteur de fermentation (2).

8. Dispositif selon la revendication 6 ou 7,
**caractérisé en ce qu'**un conduit dérivé de biogaz (6) partant du conduit de biogaz (3) débouche dans le conduit directeur (5) ou à proximité d'une extrémité ouverte du conduit directeur (5).

9. Dispositif selon l'une des revendications 6 à 8,
**caractérisé en ce que** la géométrie du dispositif conducteur (5) est choisie de manière à garantir un temps de contact suffisant entre le milieu de fermentation et le milieu contenant l'accepteur d'électrons pour inhiber sensiblement une formation de H₂S dans le milieu de fermentation.

10. Dispositif selon l'une des revendications 6 à 9,
**caractérisé en ce que** la géométrie du conduit directeur (5), la taille du réacteur de fermentation (2) et les dimensions du conduit dérivé de biogaz (6) sont choisies de manière à pouvoir transporter la totalité du milieu de fermentation contenu dans le réacteur de fermentation (2) au moins deux fois par heure à travers le conduit directeur (5), pour homogénéiser l'ensemble du contenu du réacteur de fermentation.
